Europäisches Patentamt

European Patent Office

Office européen des brevets

(11) Publication number: **0 320 519 B1**

# EUROPEAN PATENT SPECIFICATION

(45) Date of publication of patent specification: **14.10.92**

(51) Int. Cl.⁵: **A61K 31/74**, A61K 47/00

(21) Application number: **87118515.3**

(22) Date of filing: **14.12.87**

(54) **Cholestyramine compositions and method for preparation thereof.**

(43) Date of publication of application:
**21.06.89 Bulletin 89/25**

(45) Publication of the grant of the patent:
**14.10.92 Bulletin 92/42**

(84) Designated Contracting States:
**AT BE CH DE ES FR GB GR IT LI LU NL SE**

(56) References cited:
**EP-A- 0 227 603
BE-A- 896 241
FR-A- 2 193 601**

(73) Proprietor: **AMER & CO.**
**P.O. Box 125
Greenwich Connecticut 06836(US)**

(72) Inventor: **Amer, Moh. Samir**
**P.O.Box 1439
Santa Barbara, CA 93102(US)**
Inventor: **Gray, Jack C.**
**RR1, 830-1 Shady Rest Drive
Sag Harbor, NY 11963(US)**

(74) Representative: **Baillie, Iain Cameron et al**
**c/o Ladas & Parry, Altheimer Eck 2
W-8000 München 2(DE)**

**Description**

This invention relates to cholestyramine compositions and to a method for preparation of such compositions.

It is well known that various diseases are caused by high cholesterol levels in the blood plasma. Indeed, the most serious and life-threatening of these may be cardiac diseases such as arterosclerosis and coronary heart disease.

Accordingly, the art has considered it highly desirable to provide compositions, particularly for oral administration, which effectively reduce the cholesterol level in the blood.

Cholestyramine, a hydrophilic polyacrylic quaternary ammonium anion exchange resin, which is described in U.S. Patent No. 3,308,020 to Wolf et al, as well as in U.S. Patents 3,769,399 to Hagerman et al; 3,846,541 to Howard; 4,172,120 to Todd et al; 4,252,790 to Higuchi; and 4,340,585 to Borzatta et al; is well known and accepted as very effective in reducing blood cholesterol levels. It is available commercially from the Mead-Johnson division of the Bristol-Myers Company in a powder product named "Questran".

The effectiveness of cholestyramine is based on its ability to complex with bile acids. More particularly, cholesterol is probably the sole precursor of bile acids which during normal digestion are secreted into the intestines. A major portion of the bile acids is adsorbed from the intestinal tract and returned to the liver via the enterohepatic circulation. Cholestyramine adsorbs and/or reacts, binds, combines and/or complexes with the bile acids in the intestine to form an insoluble complex which is excreted in the feces. This loss of bile acids from the system leads to an increased oxidation of cholesterol to replace the loss, with a resulting decrease in beta or low density lipoprotein plasma levels and serum cholesterol levels.

Because of its low binding capacity for bile acids, the normally recommended daily dose of cholestyramine is 8 to 16 grams. This presents a major problem since the resin has an unpleasant fishy odor, gritty texture and objectionable taste properties and patients find it extremely difficult to ingest this amount of material day after day. Attempts to increase this binding capacity of cholestyramine, and thereby enable a reduction in the daily dosage, have consistently failed.

Prior attempts to mask the objectionable properties have been counter-productive. For instance, when cholestyramine is incorporated into cookies, breads, cake products such as brownies, etc., the high saturated fat and/or sugar contents of such products tend to defeat the blood cholesterol-reducing purpose of cholestyramine and/or introduce calorie-increasing and other problems.

It is an object of this invention to provide cholestyramine compositions in which the aforesaid objectionable properties, especially the fishy odor, are effectively masked or eliminated, thereby making it palatable to users.

It is a further object of this invention to provide such improved cholestyramine compositions of relatively low calorie content and/or totally or substantially devoid of saturated fat.

It is still a further object of this invention to provide a process for preparing such cholestyramine compositions.

Another object of this invention is the provision of such improved cholestyramine compositions in diverse liquid and solid oral dosage forms.

Additional objects and advantages will be apparent from a consideration of the following description.

In accordance with certain of its aspects this invention relates to an orally ingestable composition comprising cholestyramine admixed with, per part by weight, of the cholestyramine, at least one deodorizing agent that is:

0.2 to 4.5 parts by weight of a water-soluble carbohydrate-containing aqueous syrup.

Other aspects of this invention include methods for making such compositions and oral liquid and solid dosage forms thereof.

It is recognized that in U.S. Patent 3,308,020 to Wolf et al, Example 7 thereof describes a therapeutic diet for treating hypercholesteremia containing Acrysol CQ, which is disclosed as an alternative to cholestyramine, and corn oil, a water insoluble liquid fat unrelated to the aqueous syrupy carbohydrate solutions employed herein.

Similar comments apply to compositions fed to the groups of rats receiving cholestyramine in Formulation C of U.S. Patent 3,769,399 to Hagerman et al, which contains modified corn starch and corn oil, and to Example 9 of U.S. Patent 3,383,281 to Wolf et al, referring to a therapeutic diet for treating hypercholesteremia containing corn oil and a water-insoluble styrene divinylbenzene quaternary ammonium compound.

It is also recognized that U.S. Patent 4,340,585 to Borzatta et al discloses a solution of cholestyramine resin in propylene glycol for oral administration to rats. Such solution is quite different from the paste, doughy and powder compositions formed in accordance with the present invention.

EP-A-0227603 (Warner Lambert) discloses a confectionary delivery system in which the active cholestyramine is first precoated with lecithin, glycerides, polyalkylene glycols or synthetic or natural waxes. There is no disclosure of an aqueous syrup containing at least 65% of water-soluble sugar carbohydrate within a cholestyramine:syrup ratio ranging from 1:1 to 1:4.5.

Belgian 896, 241 (Prosan) discloses the use of cholestyramine for antidiarrhoea veterinarian compositions. No process is disclosed involving blending with a sugar carbohydrate containing syrup.

The water-soluble carbohydrate in the aqueous (water) syrup which suppresses the fishy odor of cholestyramine in accordance with this invention is usually and preferably a member of the sugar family. Preferred syrups include corn (preferably high fructose) syrup, sucrose syrup and invert sugar syrup. Other examples of operative water-soluble carbohydrates suitable for making such syrups include honey, molasses, glucose, maltose, dextrose, levulose, mannose and poly-dextrose. The carbohydrate in corn syrup may have a fructose content of 2 to 100% by weight. It is preferred to employ corn syrup modified to contain carbohydrate having a high fructose content of 80 - 100% by weight, most preferably 90% as in Example 1 below, in the practice of the present invention since fructose is 170% w/w sweeter than sucrose.

The carbohydrate solids content of the syrup should be sufficient to initially produce upon admixture with the cholestyramine a pasty or doughy mass, generally being at least 50 wt.%, for example 50 to 90 wt.% solids, preferably 65 to 85 wt.%, more preferably 75 to 80 wt.%, and still more preferably 77%. The amount of syrup effective for initially producing with the cholestyramine a pasty or dough mass, i.e., the syrup: cholestyramine weight ratio, is more or less dependent in any particular instance on the solids content of the syrup. Stated otherwise, the syrup solids content and the syrup:cholestyramine weight ratio are inter-related and so adjusted as to initially produce a pasty or dough mass upon admixture of the syrup with the cholestyramine. Such weight ratio may generally range from 4.5-0.2:1, preferably 3:1 to 1:1, more preferably 1.8:1 to 1.2:1, still more preferably 1.5:1.

In accordance with a preferred embodiment of this invention, the composition is prepared by blending together the appropriate ratio of cholestyramine and water-soluble carbohydrate syrup. The mixture initially formed is very sticky with a pasty or dough-like consistency. Mixing is continued to produce a light, fluffy, odorless (devoid of fishy odor) powder of homogeneous appearance. If more than light, mechanical agitation is required to produce the light, fluffy powder, the mixture may be milled, for instance in a Fitzpatrick Mill to achieve this end, and/or the above-described syrup solids content and syrup: cholestyramine weight ratios suitably adjusted.

In the practice of the present invention it is often desirable to prepare an ultimate product which supplies 2.7 gms. of cholestyramine. Such a non-odorous product is readily taken orally, 3-6 times daily in order to supply the normal daily prescription dosage of 8-16 gms. For example, prepackaged portions of the above described deodorant/cholestyramine powder containing 2.7 gms. of the cholestyramine can be readily dissolved in water or skimmed milk to provide a readily ingested odorless beverage. In general, orally ingestible products may be prepared containing any aliquot dosage portions of the deodorant/cholestyramine powder of this invention, for example units or pieces of candy each containing sufficient powder to provide 2-3 grams of cholestyramine.

The deodorized compositions can also be readily provided with color and flavor to enhance their appeal to the user. For instance color material and/or flavor may be added, preferably to the deodorant, deodorizing agent or material, that is the water-soluble carbohydrate syrup, before blending with the cholestyramine.

Typical color material are those associated with citrus fruit, e.g. orange, yellow and green. They are typically present in amounts of about 0.02 to 2.0 wt.% of the cholestyramine.

Typical flavors are those which provide mint or citrus character, e.g. peppermint, spearmint, wintergreen, clove, orange, lemon or lime. They are typically present in amounts of 0.05 to 4.0 wt.% of the cholestyramine.

The powder or beverage made therefrom may be found by some users to be somewhat "gritty" or "chalky" to the taste and feel in the mouth. This is readily and substantially overcome by including microcrystalline cellulose or a binding material (that is a gum or gum-like material) preferably with the cholestyramine prior or its mixing with the deodorizing material. Such binding materials are typically cellulose binders such as methyl cellulose, hydroxyethyl cellulose, hydroxypropyl methyl cellulose, cellulose esters, sodium carboxymethyl cellulose, etc., or polysaccharides such as the xanthamonas colloid, xanthan. The microcrystalline cellulose or binder is typically employed in amounts of 3 to 30% by weight of the cholestyramine, preferably 5 to 25%. Besides reducing grittiness or chalkiness, these additives may also reduce the tendency some users have toward constipation.

Moreover, as desired, further additives may be blended into the powder of types and in amounts well accepted in the art. Such further additives may include artificial sweeteners such as sodium saccharin, sodium cyclamate and Aspartame, as well as vitamins and minerals.

The deodorized composition of the present invention is also desirably formulated and/or shaped into unitary products such as granules, tablets, wafers and cookie shapes.

In order to make such unitary solid consumable product from the above-described deodorant/cholestyramine odorless powder, water-soluble carbohydrate syrup as described above, in proportions sufficient to produce a "sheetable" dough, is blended into the powder in typical weight ratios of such carbohydrate syrup to deodorized powder of 0.5-1.5:1, preferably 0.8:1 to 1:1. Although any normally consumable water-soluble carbohydrate syrup may be used in making the unitary cholestyramine product, polydextrose syrup liquid is preferred because of its relatively low caloric nature and since relatively less of it is generally needed to produce the desired sheetable dough mass, for example polydextrose syrup:deodorized powder weight ratios of 0.4:1 to 0.7:1, preferably 0.5:1 to about 0.65:1.

Polydextrose, produced as described in U.S. 3,766,165 (1973) and 3,876,794 (1975), is a water soluble randomly bonded condensation polymer of dextrose containing minor amounts of bonded sorbitol and citric acid. Commercially available polydextrose Type N aqueous syrup (Pfizer) contains about 70% polydextrose of which 88.7% has a molecular weight (M.W.) range of about 162 to 5,000, 10% has a M.W. of about 5,000 to 10,000, 1.2% has a M.W. range of about 10,000 to 16,000 and 0.1% has a M.W. range of about 16,000 to 18,000.

After blending of polydextrose syrup or the like carbohydrate syrup with the deodorized powder, a dough readily forms which can be formed, rolled, pressed or extruded into sheets. Such sheets can then be sliced or rolled more thinly and cut into any desired shapes, such as wafers which provide a dosage amount of cholestyramine.

When wafers are formed in accordance with the present invention, for instance measuring .3175-.635cm (1/8-1/4 inch) in thickness and 3.81-6.35cm (1.5-2.5 inches) in average diameter, they possess sufficient cohesiveness and structural strength to be packaged and shipped for ultimate consumption.

Structural strength and cohesiveness of wafers may be further increased by the presence in the deodorized powder of the binding gums or gum-like materials discussed above as well as water dispersible proteins such as vital wheat gluten, sodium caseinate, egg albumin or soy isolate. Typically the weight ratio of such cohesiveness-increasing additive to cholestyramine is about 0.05-0.5:1.

Moreover, structural strength and cohesiveness of wafers can also be increased by coating the wafer product in industrially recognized manners or by sandwiching the wafer as a filling between previously formed solid pieces of ingestible material.

The following Examples are only illustrative of the invention and not limitative. All amounts and proportions referred to herein and in the appended claims are by weight and temperatures are in degrees C° [F.] unless otherwise indicated.

## EXAMPLE 1

Mixture A and Mixture B are prepared:

| Mixture A | |
|---|---|
| Cholestyramine | 8.0 parts |
| Methyl Cellulose (Dow A151V) | 1.0 parts |
| Xanthan Gum | 0.7 parts |

| Mixture B | |
|---|---|
| 90% High Fructose Corn Syrup (77%) solids | 12.0 parts |
| Liquid Orange Flavor | 0.1 parts |
| Liquid Orange Color | 0.07 parts |

Mixtures A and B are combined in a Hobart A-120 vertical mixer with a paddle, blended for 5 minutes, the sides are scraped down with the paddle and then blending is continued for an additional 10 minutes. The mixture is sticky and essentially dough-like in consistency at first but turns to a light and fluffy powder with no unpleasant odor.

Next 13.5 parts of polydextrose Type N liquid (Pfizer) are added and blended for 1 minute until a dough is formed. The sides are scraped down with the paddle and the ingredients are mixed until they are uniformly dispersed throughout the dough.

The dough is then rolled out to a uniform thickness of .476cm (3/16") using a rolling pin. In some preparations a small amount of starch is added should sticking occur. Wafers are then cut out using a "Hex" cutter measuring 4.921cm (1 15/16") (flat side to flat side) Each wafer weighs 11.8 grams and three wafers contain the normal daily dosage of 8.0 grams of cholestyramine.

EXAMPLE 2

Mixture A and Mixture B are prepared:

| Mixture A | |
| --- | --- |
| Cholestyramine | 8.0 grams |
| FMC Microcrystalline Cellulose (Type B CL-611) | 0.5 grams |
| Aspartame | 0.05 grams |

| Mixture B | |
| --- | --- |
| Glycerine (99.5%) | 4.8 grams |
| Liquid Lemon Lime | 0.1 grams |
| Liquid Mint Green | 0.05 grams |

Mixture A is blended with Mixture B in a 2509 ml. glass beaker with a spatula. The mixture is paste-like at first but becomes a light and fluffy powder with no unpleasant odor as blending is continued. The deodorized light fluffy powder is added to one cup of cold tap water to produce a desirable, readily ingestible beverage containing the normal daily dosage of 8 grams of cholestyramine.

EXAMPLE 3

Mixture A and Mixture B are prepared:

| Mixture A | | Parts |
| --- | --- | --- |
| Cholestyramine | | 8.0 |
| Xanthan gum | | 0.7 |
| Vital Wheat Gluten | | 1.2 |
| Butter/Vanilla/Lemon Flavor | | 0.25 |

| Mixture B | | Parts |
| --- | --- | --- |
| Invert Sugar Aqueous Syrup (77%) solids | | 12.0 |
| Liquid Yellow | | 0.01 |

As in Example 1, Mixtures A and B are combined, blended with 13.5 parts of polydextrose liquid and the resultant deodorized dough rolled out and cut into wafers.

Each wafer is dipped into the following mixture:

|  | Parts |
|---|---|
| Unbleached Pastry Flour | 59.9 |
| Polydextrose Powder | 30.0 |
| Safflower Oil | 8.0 |
| Potassium Bicarbonate | 1.0 |
| Anhydrous Monocalcium Phosphate | 1.1 |
| Water | 45.0 |

The dipped wafers are placed on a parchment lined baking pan and baked for 6 minutes at 204°C (400°F.)

Alternatively, instead of dipping into the above mixture, each wafer is dipped in a chocolate coating mixture and heated to 46°C (115°F), drained and then cooled to set the chocolate coating.

Readily ingestible, tasty coated wafers are thus produced containing incremental dosage amounts of the cholestyramine as normally prescribed.

**Claims**

**Claims for the following Contracting States : AT, BE, CH, DE, FR, GB, IT, LI, LU, NL, SE**

1. An orally ingestible cholestyramine containing composition comprising cholestyramine and an aqueous syrup containing at least 65% of water-soluble sugar carbohydrate at a cholestyramine:syrup ratio ranging from 1:1 to 1:4.5.

2. A composition according to Claim 1, characterized in that the water-soluble carbohydrate-containing syrup consists of corn syrup, sucrose syrup, invert sugar syrup or polydextrose syrup.

3. A composition according to Claim 1 characterized in that the syrup is high fructose corn syrup.

4. A composition according to either of Claims 1, 2 and 3 characterized by being in the form of:
   (a) a paste or dough;
   (b) a powder derived from (a);
   (c) a wafer or other solid shaped form made from (a) or (b); or
   (d) an aqueous beverage containing a dosage amount of said powder.

5. A composition according to any of Claims 1 - 4, characterized by further containing;
   (1) an amount of xanthan gum or microcrystalline cellulose sufficient to reduce grittiness of solid shaped products derived from the paste or dough, to an acceptable level or
   (2) an amount of a binding material consisting of methyl cellulose, hydroxyethyl cellulose, hydroxypropyl methyl cellulose, carboxymethyl cellulose or a polysaccharide sufficient to reduce the grittiness to an acceptable level and/or improve structural strength of solid shaped products derived from the paste or dough.

6. A method of making an orally ingestible cholestyramine containing composition, characterized by blending with the cholestyramine an amount of an aqueous syrup containing at least 65% of water-soluble sugar carbohydrate at a cholestyramine:syrup ratio ranging from 1:1 to 1:4.5 sufficient to form a paste or dough.

7. A method according to Claim 6 characterized in that the water-soluble carbohydrate-containing syrup consists of corn syrup, high fructose corn syrup, sucrose syrup, invert sugar syrup or polydextrose syrup.

8. A method according to either of Claims 6 and 7 characterized by further adding (1) an amount of xanthan gum or microcrystalline cellulose sufficient to reduce grittiness of solid shaped products derived from the resulting paste or dough to an acceptable level or (2) an amount of a binding material consisting of methyl cellulose, hydroxyethyl celluloe, hydroxypropyl methyl cellulose, carboxymethyl cellulose or a polysaccharide sufficient to reduce grittiness to an acceptable level and/or improve structural strength of solid shaped products derived from the paste or dough.

6

**9.** The method according to either of Claims 6 and 7 followed by further mixing the paste or dough to form a substantially odorless powder.

**10.** The method according to Claim 9 characterized by blending the powder with sufficient water soluble carbohydrate-containing syrup to form a doughy mass.

**11.** The method according to Claim 10 characterized in that the syrup blended with the powder contains polydextrose as the water soluble carbohydrate.

**12.** A wafer or other solid shaped orally ingestible product derived from the product produced by the method of any of Claims 6, 7, 8, 9, 10 or 11.

**Claims for the following Contracting States : ES, GR**

**1.** A method of making an orally ingestible cholestyramine containing composition, characterized by blending with the cholestyramine an amount of an aqueous syrup containing at least 65% of water-soluble sugar carbohydrate at a cholestyramine:syrup ratio ranging from 1:1 to 1:4.5 sufficient to form a paste or dough.

**2.** A method according to Claim 1 characterized in that the water-soluble carbohydrate-containing syrup consists of corn syrup, high fructose corn syrup, sucrose syrup, invert sugar syrup or polydextrose syrup.

**3.** A method according to either of Claims 1 and 2 characterized by further adding (1) an amount of xanthan gum or microcrystalline cellulose sufficient to reduce grittiness of solid shaped products derived from the resulting paste or dough to an acceptable level or (2) an amount of a binding material consisting of methyl cellulose, hydroxyethyl cellulose, hydroxypropyl methyl cellulose, carboxymethyl cellulose or a polysaccharide sufficient to reduce grittiness to an acceptable level and/or improve structural strength of solid shaped products derived from the paste or dough.

**4.** The method according to either of Claims 1 and 2 followed by further mixing the paste or dough to form a substantially odorless powder.

**5.** The method according to either of Claims 1 and 2 characterized by blending the powder with sufficient water soluble carbohydrate-containing syrup to form a doughy mass.

**6.** The method according to Claim 5 characterized in that the syrup blended with the powder contains polydextrose as the water soluble carbohydrate.

**Patentansprüche**
**Patentansprüche für folgende Vertragsstaaten : AT, BE, CH, DE, FR, GB, IT, LI, LU, NL, SE**

**1.** Oral einnehmbare Cholestyramin enthaltende Zusammensetzung umfassend Cholestyramin und einen wässerigen Sirup enthaltend wenigstens 65 % wasserlöslichen Kohlenhydratzucker bei einem Cholestyramin : Sirup-Verhältnis im Bereich von 1:1 bis 1:4,5.

**2.** Zusammensetzung nach Anspruch 1, dadurch gekennzeichnet, daß der wasserlösliches Kohlenhydrat enthaltende Sirup aus Stärkesirup, Saccharosesirup, Invertzuckersirup oder Polydextrosesirup besteht.

**3.** Zusammensetzung nach Anspruch 1, dadurch gekennzeichnet, daß der Sirup Stärkesirup mit hohem Fructosegehalt ist.

**4.** Zusammensetzung nach einem der Ansprüche 1 bis 3, dadurch gekennzeichnet, daß sie in Form
(a) einer Paste oder eines Teigs;
(b) eines aus (a) erhaltenen Pulvers;
(c) einer Oblate oder einer anderen festen geformten aus (a) oder (b) hergestellten Form oder
(d) eines wässerigen Getränks enthaltend eine Dosismenge des erwähnten Pulvers vorliegt.

**5.** Zusammensetzung nach einem der Ansprüche 1 bis 4, dadurch gekennzeichnet, daß sie weiters
(1) eine zur Verringerung der Sandigkeit von aus der Paste oder dem Teig erhaltenen festen geformten Produkten auf ein annehmbares Ausmaß ausreichende Menge von Xanthangummi oder mikrokristalliner Cellulose oder
(2) eine zur Verringerung der Sandigkeit auf ein annehmbares Ausmaß und/oder Verbesserung der strukturellen Festigkeit von aus der Paste oder dem Teig erhaltenen festen geformten Produkten ausreichende Menge eines Bindematerials, bestehend aus Methylcellulose, Hydroxyethylcellulose, Hydroxypropylmethylcellulose, Carboxymethylcellulose oder einem Polysaccharid, enthält.

**6.** Verfahren zur Herstellung einer oral einnehmbaren Cholestyramin enthaltenden Zusammensetzung, dadurch gekennzeichnet, daß man mit dem Cholestyramin eine zur Bildung einer Paste oder eines Teigs ausreichende Menge eines wässerigen Sirups enthaltend wenigstens 65 % wasserlöslichen Kohlenhydratzucker bei einem Cholestyramin : Sirup-Verhältnis im Bereich von 1:1 bis 1:4,5 vermischt.

**7.** Verfahren nach Anspruch 6, dadurch gekennzeichnet, daß der wasserlösliches Kohlenhydrat enthaltende Sirup aus Stärkesirup, Stärkesirup mit hohem Fructosegehalt, Saccharosesirup, Invertzuckersirup oder Polydextrosesirup besteht.

**8.** Verfahren nach Anspruch 6 oder 7, dadurch gekennzeichnet, daß man zusätzlich (1) eine zur Verringerung der Sandigkeit von aus der erhaltenen Paste oder dem Teig erhaltenen festen geformten Produkten auf ein annehmbares Ausmaß ausreichende Menge von Xanthangummi oder mikrokristalliner Cellulose oder (2) eine zur Verringerung der Sandigkeit auf ein annehmbares Ausmaß und/oder Verbesserung der strukturellen Festigkeit von aus der Paste oder dem Teig erhaltenen festen geformten Produkten ausreichende Menge eines Bindematerials, bestehend aus Methylcellulose, Hydroxyethylcellulose, Hydroxypropylmethylcellulose, Carboxymethylcellulose oder einem Polysaccharid, zusetzt.

**9.** Verfahren nach Anspruch 6 oder 7, gefolgt vom weiteren Mischen der Paste oder des Teigs zur Bildung eines im wesentlichen geruchlosen Pulvers.

**10.** Verfahren nach Anspruch 9, gekennzeichnet durch Mischen des Pulvers mit ausreichend wasserlösliches Kohlenhydrat enthaltendem Sirup zur Bildung einer teigigen Masse.

**11.** Verfahren nach Anspruch 10, dadurch gekennzeichnet, daß der mit dem Pulver vermischte Sirup Polydextrose als wasserlösliches Kohlenhydrat enthält.

**12.** Oblate oder ein anderes festes geformtes oral einnehmbares Produkt, erhalten von dem durch das Verfahren nach einem der Ansprüche 6, 7, 8, 9, 10 oder 11 hergestellten Produkt.

**Patentansprüche für folgende Vertragsstaaten : ES, GR**

**1.** Verfahren zur Herstellung einer oral einnehmbaren Cholestyramin enthaltenden Zusammensetzung, dadurch gekennzeichnet, daß man mit dem Cholestyramin eine zur Bildung einer Paste oder eines Teigs ausreichende Menge eines wässerigen Sirups enthaltend wenigstens 65 % wasserlöslichen Kohlenhydratzucker bei einem Cholestyramin : Sirup-Verhältnis im Bereich von 1:1 bis 1:4,5 vermischt.

**2.** Verfahren nach Anspruch 1, dadurch gekennzeichnet, daß der wasserlösliches Kohlenhydrat enthaltende Sirup aus Stärkesirup, Stärkesirup mit hohem Fructosegehalt, Saccharosesirup, Invertzuckersirup oder Polydextrosesirup besteht.

**3.** Verfahren nach Anspruch 1 oder 2, dadurch gekennzeichnet, daß man zusätzlich (1) eine zur Verringerung der Sandigkeit von aus der erhaltenen Paste oder dem Teig erhaltenen festen geformten Produkten auf ein annehmbares Ausmaß ausreichende Menge von Xanthangummi oder mikrokristalliner Cellulose oder (2) eine zur Verringerung der Sandigkeit auf ein annehmbares Ausmaß und/oder Verbesserung der strukturellen Festigkeit von aus der Paste oder dem Teig erhaltenen festen geformten Produkten ausreichende Menge eines Bindematerials, bestehend aus Methylcellulose, Hydroxyethylcellulose, Hydroxypropylmethylcellulose, Carboxymethylcellulose oder einem Polysaccharid, zusetzt.

**4.** Verfahren nach Anspruch 1 oder 2, gefolgt vom weiteren Mischen der Paste oder des Teigs zur Bildung eines im wesentlichen geruchlosen Pulvers.

5. Verfahren nach Anspruch 1 oder 2, gekennzeichnet durch Mischen des Pulvers mit ausreichend wasserlösliches Kohlenhydrat enthaltendem Sirup zur Bildung einer teigigen Masse.

6. Verfahren nach Anspruch 5, dadurch gekennzeichnet, daß der mit dem Pulver vermischte Sirup Polydextrose als wasserlösliches Kohlenhydrat enthält.

**Revendications**

**Revendications pour les Etats contractants suivants : AT, BE, CH, DE, FR, GB, IT, LI, LU, NL, SE**

1. Composition contenant de la cholestyramine ingérable par voie orale, comprenant de la cholestyramine et un sirop aqueux contenant au moins 65% d'un glucide sucré hydrosoluble dans un rapport chlolestyramine : sirop compris entre 1 : 1 et 1 : 4,5.

2. Composition selon la revendication 1, caractérisée en ce que le sirop contenant le glucide hydrosoluble est constitué par un sirop de maïs, un sirop de saccharose, un sirop de sucre inverti ou un sirop de polydextrose.

3. Composition selon la revendication 1, caractérisée en ce que le sirop est un sirop de maïs à teneur élevée en fructose.

4. Composition selon l'une quelconque des revendications 1, 2 et 3, caractérisée en ce qu'elle se présente sous la forme de :
   **(a)** une pâte;
   **(b)** une poudre dérivée de **(a)**;
   **(c)** un cachet ou une autre forme solide façonnée préparée à partir de **(a)** ou **(b)** ; ou
   **(d)** une boisson aqueuse contenant une dose de ladite poudre.

5. Composition selon l'une quelconque des revendications 1 à 4, carac-térisée en ce qu'elle contient en outre :
   (1) un quantité de gomme de xanthanne ou de cellulose microcristalline suffisante pour réduire le caractère sableux des produits solides façonnés dérivés de la pâte, à un niveau acceptable ou
   (2) une quantité d'un liant constitué par la méthylcellulose, l'hydroxy-éthylcellulose, l'hydroxypropylméthylcellulose, la carboxyméthylcellulose ou un polysaccharide, suffisante pour réduire le caractère sableux à un niveau acceptable et/ou améliorer la résistance structurale des produits solides façonnés dérivés de la pâte.

6. Procédé de préparation d'une composition contenant de la cholestérine ingérable par voie orale, caractérisé par le mélange avec la cholestyramine d'une quantité d'un sirop aqueux contenant au moins 65% de glucide sucré hydrosoluble dans un rapport cholestyramine : sirop compris entre 1 : 1 et 1 : 4,5, suffisante pour former une pâte.

7. Procédé selon la revendication 6, caractérisé en ce que le sirop contenant le glucide hydrosoluble est constitué par un sirop de maïs, un sirop de maïs à forte teneuren fructose, un sirop de saccharose, un sirop de sucre inverti ou un sirop de polydextrose.

8. Procédé selon l'une quelconque des revendications 6 et 7, caractérisé en ce qu'on ajoute en outre (1) une quantité de gomme de xanthanne ou de cellulose microcristalline suffisante pour réduire le caractère sableux des produits solides façonnés dérivés de la pâte obtenue à un niveau acceptable ou (2) une quantité d'un liant constitué par la méthylcellulose, l'hydroxyéthylcellulose, l'hydroxypropylméthylcellulose, la carboxyméthylcellulose ou un polysaccharide, suffisante pour réduire le caractère sableux à un niveau acceptable et/ou améliorer la résistance structurale des produits solides façonnés dérivés de la pâte.

9. Procédé selon l'une quelconque des revendications 6 et 7, suivi par la poursuite du mélange de la pâte pour former une poudre sensiblement inodore.

**10.** Procédé selon la revendication 9, caractérisé par le mélange de la poudre avec une quantité suffisante de sirop contenant un glucide hydrosoluble pour former une masse pâteuse.

**11.** Procédé selon la revendication 10, caractérisé en ce que le sirop mélange à la poudre contient du polydextrose comme glucide hydrosoluble.

**12.** Cachet ou autre produit ingérable par voie orale, solide, façonné, dérivé du produit préparé selon le procédé de l'une quelconque des revendications 6, 7, 8, 9, 10 ou 11.

**Revendications pour les Etats contractants suivants : ES, GR**

**1.** Procédé de préparation d'une composition contenant de la chlolestyramine ingérable par voie orale, caractérisé par le mélange avec la cholestyramine d'une quantité d'un sirop aqueux contenant au moins 65% de glucide sucré hydrosoluble dans un rapport cholestyramine : sirop compris entre 1 : 1 et 1 : 4,5, suffisante pour former une pâte.

**2.** Procédé selon la revendication 1, caractérisé en ce que le sirop contenant un glucide hydrosoluble est constitué par un sirop de maïs, un sirop de maïs à forte teneur en fructose, un sirop de saccharose, un sirop de sucre inverti ou un sirop de polydextrose.

**3.** Procédé selon l'une des revendications 1 et 2, caractérisé en ce qu'on ajoute en outre (1) une quantité de gomme de xanthanne ou de cellulose microcristalline suffisante pour réduire le caractère sableux des produits solides façonnés dérivés de la pâte obtenue à un niveau acceptable ou (2) une quantité d'un liant constituée par la méthylcellulose, l'hydroxyéthylcellulose, l'hydroxypropylméthylcellulose, la carboxyméthylcellulose ou un polysaccharide, suffisante pour réduire le caractère sableux à un niveau acceptable et/ou améliorer la résistance structurale de produits solides façonnés dérivés de la pâte.

**4.** Procédé selon l'une des revendications 1 et 2, suivi par la poursuite du mélange de la pâte pour former une poudre sensiblement inodore.

**5.** Procédé selon l'une quelconque des revendications 1 et 2, caractérisé par le mélange de la poudre avec suffisamment de sirop contenant un glucide hydrosoluble pour former une masse pâteuse.

**6.** Procédé selon la revendication 5, caractérisé en ce que le sirop mélangé à la poudre contient du polydextrose comme glucide hydrosoluble.